# EUROPÄISCHE PATENTANMELDUNG

(11) **EP 2 338 539 A1**
(43) Veröffentlichungstag der Anmeldung: **29.06.2011**
(21) Anmeldenummer: 09075570.3
(22) Anmeldetag: 23.12.2009
(51) Int. Cl.: A61M 1/10, F04D 3/00

(54) **Pumpeneinrichtung mit einer Detektionseinrichtung**

(71) Anmelder: ECP Entwicklungsgesellschaft mbH, 12247 Berlin (DE)
(72) Erfinder: Schumacher, Jörg, 14513 Teltow (DE)
(74) Vertreter: Pfenning, Meinig & Partner GbR

(57) **Zusammenfassung**

Die Erfindung bezieht sich auf eine Pumpeneinrichtung mit einer Pumpe (8) und einer Energiezuführungseinrichtung (5, 18), wobei die Pumpe ein Förderelement (9, 11) aufweist, das mittels zugeführter Energie ein Fluid fördert, wobei die Pumpe einen Transportzustand und einen Betriebszustand aufweist und wobei wenigstens ein erstes Element (9, 9a, 10, 10', 11) der Pumpe im Transportzustand eine andere Form und/oder Größe aufweist als im Betriebszustand. Die Betriebssicherheit einer solchen Pumpeneinrichtung wird erhöht durch eine Detektionseinrichtung (12, 20, 21, 22, 23, 24, 25, 27, 28, 29), die mittels eines Sensors erfasst, ob wenigstens das erste Element sich bezüglich Form und/oder Größe im Betriebszustand befindet.

## Beschreibung

Die Erfindung liegt auf dem Gebiet des Maschinenbaus und der Feinwerktechnik und betrifft eine Pumpeneinrichtung.

Pumpen werden zur Förderung von Fluiden, insbesondere von Flüssigkeiten eingesetzt und sind in vielfältigen Varianten bekannt. Besonders interessant für viele Anwendungsfälle sind Pumpen, die entweder eine besonders kleine Bauart aufweisen oder in ihrer Gestalt veränderbar sind, um in einem Transportzustand an einen schwierig zugänglichen Einsatzort gebracht zu werden, wobei danach ein Betriebszustand herstellbar ist, in dem einzelne Elemente der Pumpe eine andere Form und/oder Größe aufweisen können als im Betriebszustand.

Beispiele für derartige Pumpen sind entfaltbare Katheterpumpen, die durch ein Blutgefäß in einen Patientenkörper eingeführt und beispielsweise bis in eine Herzkammer eingeschoben werden und dort entfaltet werden können.

Dazu findet typischerweise eine Entfaltung eines Rotors der Pumpe und eines Gehäuses statt. Der Rotor trägt üblicherweise ein oder mehrere Schaufelblätter, die allein oder gemeinsam mit einer Nabe radial komprimierbar und expandierbar sind. Zu diesem Zweck können Förderschaufeln beispielsweise biegbar oder schwenkbar an einer Nabe befestigt oder in sich durch die Herstellung als Schaumstoffkörper kompressibel ausgebildet sein. Gelegentlich weisen solche Förderschaufeln auch Anteile aus einer Gedächtnislegierung wie beispielsweise Nitinol auf, die superelastische Eigenschaften und temperaturabhängige Formeigenschaften aufweist. Ein entsprechender Rotor ist dann mit geringer Kraft radial komprimierbar und richtet sich bei geeigneter Temperatur nach Wegfallen der Kompressionskraft selbständig wieder zur ursprünglichen Gestalt auf.

Es sind auch Prinzipien bekannt, nach denen eine entsprechende Pumpe durch Betätigung von außen nach dem Transport zum Einsatzort aktiv aufgerichtet/expandiert wird (WO 94/05347 A1). Derartige Konstruktionen benötigen jedoch entsprechende Betätigungseinrichtungen und Elemente zur Übertragung einer solchen Betätigung, was einen erhöhten elektrischen, pneumatischen oder mechanischen Aufwand erfordert.

Es sind auch Pumpenprinzipien bekannt, bei denen Förderschaufeln eines Rotors sich bei Inbetriebnahme durch den Fluidgegendruck mit wachsender Geschwindigkeit des Rotors bis zu einem Betriebszustand aufrichten, in dem der Rotor maximal in radialer Richtung aufgeweitet ist.

Allen genannten Prinzipien ist gemein, dass nach dem Verbringen der Pumpe an den Einsatzort eine Expansion der Pumpenelemente eingeleitet oder ermöglicht wird, dass jedoch das Erreichen des Betriebszustandes nicht absolut sicher ist. Somit besteht die Gefahr, dass trotz entsprechender Betätigung die Pumpe den Betriebszustand gar nicht oder nur teilweise erreicht. Darin liegt insbesondere bei Einsatz derartiger Pumpen im biologischen Bereich eine große Gefahr, da bei Inbetriebnahme der Pumpe entweder Körperflüssigkeiten wie beispielsweise Blut geschädigt werden oder bei Beschädigung der Pumpe Pumpenelemente in eine Körperflüssigkeit wie z. B. Blut hineingelangen können.

Dies kann beispielsweise geschehen, wenn ein Pumpenrotor in Rotation versetzt wird, ohne dass er seine Betriebsform aufweist oder ohne dass das ihn umgebende Gehäuse bereits auf den Solldurchmesser expandiert worden ist. In diesem Fall können Förderschaufeln brechen oder ungewollt im Transportzustand betrieben werden, so dass aufgrund eines zu geringen Fluidwiderstandes der Rotor eine zu hohe Drehzahl entwickelt und Bestandteile des Blutes oder einer anderen Körperflüssigkeit schädigt. Ist die Drehzahl gesteuert oder geregelt, so würde die Pumpe möglicherweise mit nicht ausreichender Förderleistung arbeiten.

Der vorliegenden Erfindung liegt vor dem Hintergrund des Standes der Technik die Aufgabe zugrunde, eine Pumpeneinrichtung derart auszubilden, dass Nachteile durch einen Betrieb der Pumpe außerhalb des Betriebszustandes vermieden werden.

Die Aufgabe wird gemäß der Erfindung mit den Merkmalen des Patentanspruchs 1 gelöst.

Dabei ist vorgesehen, dass die Pumpeneinrichtung eine Pumpe und eine Energiezuführungseinrichtung aufweist, wobei die Pumpe ein Förderelement aufweist, das mittels zugeführter Energie ein Fluid fördert, wobei die Pumpe einen Transportzustand und einen Betriebszustand aufweist und wobei wenigstens ein erstes Element der Pumpe im Transportzustand eine andere Form und/oder Größe aufweist als im Betriebszustand. Dabei ist zudem eine Detektionseinrichtung vorgesehen, die erfasst, ob wenigstens das erste Element sich bezüglich Form und/oder Größe im Betriebszustand befindet.

Das erste Element weist im Transportzustand eine andere Form und/oder Größe auf als im Betriebszustand, damit die Pumpe Verformungsmöglichkeiten aufweist und an einen zu durchlaufenden Transportweg angepasst werden kann, um zum Einsatzort zu gelangen. Das erste Element kann beispielsweise eine Förderschaufel, ein Rotor oder ein Pumpengehäuse sein.

Es kann beispielsweise vorgesehen sein, dass wenigstens ein erstes Element wenigstens teilweise aus einem elastisch verformbaren, insbesondere komprimierbaren und expandierbaren Material besteht. Dadurch kann das wenigstens eine erste Element den Wechsel vom Transportzustand in den Betriebszustand durch elastische Verformung, wie beispielsweise Biegen, Kompression, Extraktion oder Ähnliche Effekte vollziehen.

Beispielsweise kann eine derartige Pumpe durch ein Schlauch- Rohr- oder Kanalsystem schiebbar sein, indem sie zunächst durch radiale Kompression reduziert und später am Einsatzort wieder expandiert wird. Insbesondere werden solche Prinzipien bei medizinisch eingesetzten Mikropumpen verwirklicht, bei denen die Pumpe selbst und gegebenenfalls das Gehäuse zum Transport radial komprimiert werden.

Es ist sinnvoll, vor der Inbetriebnahme der Pumpe sicherzustellen, dass das erste Element den Betriebszustand erreicht hat. Beispielsweise kann das erste Element ein Rotor der Pumpe mit wenigstens einer Förderschaufel sein, wobei der Rotor die erforderliche Pumpleistung erst dann erreicht, wenn er entsprechend expandiert ist. Ist der Rotor von einem Gehäuse umgeben, so ist sicherzustellen, dass vor der Inbetriebnahme das Gehäuse expandiert ist. Entsprechend kann die Detektionseinrichtung den Entfaltungszustand des Gehäuses und/oder des Rotors oder einer Förderschaufel überwachen. Dies kann beispielsweise dadurch geschehen, dass die Detektionseinrichtung als Sensor einen Dehnungsmessstreifen enthält, der an einer Förderschaufel befestigt ist, oder einen Positionssensor, der beispielsweise die Relativposition von Förderschaufeln zu einer Nabe überwachen kann.

Wird eine derartige Pumpe zu früh in Betrieb gesetzt, ohne dass der Betriebszustand erreicht ist, so würde riskiert werden, dass die Förderschaufeln sich am Pumpengehäuse oder an anderen Stellen verhaken und gegebenenfalls abbrechen, in jedem Fall jedoch die Pumpe abgebremst wird.

Es kann auch geschehen, dass wegen einer ungenügenden Expansion des Rotors dieser nicht korrekt arbeitet und dass seine Förderleistung nicht ausreicht.

Die Energiezuführungseinrichtung kann vorteilhaft als mechanischer Antrieb und das Förderelement beweglich und mittels des Antriebs antreibbar ausgebildet sein. Der typische Fall ist damit durch einen drehend antreibbaren Pumpenrotor mit Förderschaufeln gegeben.

Es sind jedoch auch andere Pumpenfunktionsprinzipien denkbar, beispielsweise mit einem abwechselnd expandierten und komprimierten Ballonelement, das aus einer Kammer intermittierend Flüssigkeit verdrängt und diese damit fördert. Zudem sind auch Pumpenprinzipien ohne bewegte Teile denkbar, bei denen das Förderelement beispielsweise ein elektrisches oder magnetisches Feld erzeugt, das auf einen Flüssigkeit wirkt, die entsprechende Materialparameter aufweist, um durch entsprechende Feldeffekte gefördert werden zu können.

Auch bei derartigen nicht bewegten Förderelementen kann es sinnvoll sein, deren Entfaltungszustand zu detektieren.

Vorteilhaft kann ein Förderelement aufpumpbar, biegbar, schwenkbar oder elastisch komprimierbar und expandierbar sein. Dies gelingt beispielsweise dadurch, dass das Förderelement ganz oder in Teilen aus einem elastischen Schaumstoff besteht oder dass ein elastischer Drahtrahmen vorgesehen ist, über den zur Ausbildung einer Förderschaufel eine Membran gespannt ist, wobei der Rahmen elastisch in radialer Richtung komprimierbar ist und bei Wegfallen einer Kompressionskraft selbsttätig die ursprüngliche Form annimmt. Eine entsprechende Förderschaufel kann auch als abklappbarer oder abbiegbarer Körper an der Nabe anliegen und erst zu Beginn des Betriebes durch den sich einstellenden Fluidgegendruck aufgestellt werden.

Es kann auch zusätzlich zu einem Förderelement bzw. einem Rotor ein ebenfalls komprimierbares Gehäuse der Pumpe vorgesehen sein.

In diesem Fall ist es besonders wichtig, dass vor der Inbetriebnahme sowohl das Förderelement, beispielsweise ein Rotor, als auch das Pumpengehäuse entsprechend in den Betriebszustand versetzt werden. Dies kann durch die oben genannten Effekte entweder selbsttätig geschehen, wenn die Pumpe beim Transport radial zusammengehalten und nach dem Transport der Zusammenhalt gelöst wird. Dies kann beispielsweise dadurch realisiert sein, dass die Pumpe während des Transports in einem Hohlkatheter geführt ist und nach dem Transport oder zum Ende des Transports aus dem Hohlkatheter hinausgeschoben wird, so dass die radiale Haltekraft außerhalb des Hohlkatheters wegfällt und die Pumpe sich selbsttätig elastisch entfaltet.

Es kann jedoch auch eine aktive Entfaltung durch eine entsprechende Betätigungseinrichtung wie beispielsweise kleine Hebel oder andere Vorrichtungen, die an dem Rotor und dem Gehäuse angreifen, vorgesehen sein.

Vorteilhaft ist vorgesehen, dass die Detektionseinrichtung einen Signalzustand ändert, wenn das erste Element den Betriebszustand erreicht. Damit kann einer die Pumpe bedienenden Person der Zustand der Pumpe aktiv übermittelt werden. Beispielsweise kann der Zustand durch ein optisches oder akustisches Signal mitgeteilt werden.

Es kann auch vorgesehen sein, dass die Detektionseinrichtung ein Signal abgibt, solange der Betriebszustand nicht erreicht ist. Das entsprechende Signal kann damit als Warnsignal dienen, dass die Pumpe noch nicht in Betrieb genommen werden kann.

Wird ein Signal nur dann abgegeben, wenn der Betriebszustand erreicht ist, so ist dadurch sichergestellt, dass bei einem Defekt der Detektionseinrichtung, solange kein Signal abgegeben wird, sicherheitshalber die Pumpe nicht in Betrieb genommen wird, auch wenn sie tatsächlich bei defektem Sensor bereits den Betriebszustand erreicht haben sollte.

Es kann auch vorgesehen sein, dass die Detektionseinrichtung die Energiezuführungseinrichtung blockiert, solange der Betriebszustand nicht erreicht ist. Damit ist automatisch und ohne Zutun einer Bedienperson sichergestellt, dass die Pumpe nicht in Betrieb genommen wird, solange die Entfaltung der entfaltbaren Elemente nicht bestätigt wird.

Vorteilhaft kann auch vorgesehen sein, dass die Detektionseinrichtung im Betrieb die Last der Energiezuführungseinrichtung überwacht und durch Vergleich mit einem Referenzwert oder einem Referenzmuster ermittelt, ob der Betriebszustand erreicht ist.

Der Sensor der Detektionseinrichtung kann somit, auch entfernt von der Pumpe, beispielsweise in der Nähe der Energiezuführungseinrichtung, angeordnet sein, um von dort zu überwachen, ob durch ein unregelmäßiges Verhalten der Energiezuführungseinrichtung auf einen erhöhten oder geänderten Widerstand beim Pumpbetrieb geschlossen werden kann. Im Gegensatz dazu kann auch vorgesehen sein, dass die Detektionseinrichtung einen Sensor aufweist, der unmittelbar an der Pumpe angeordnet ist.

Dort lassen sich die Elemente der Pumpe wesentlich einfacher direkt überwachen, wenn entsprechende Sensoren wie beispielsweise Dehnungsmessstreifen vorgesehen werden. Es kann dann auch unabhängig vom Betrieb der Pumpe ermittelt werden, ob diese bereits den Betriebszustand erreicht hat, d. h. entsprechend expandiert worden ist. Beispielsweise kann die Form der einzelnen Förderschaufeln durch aufgebrachte Dehnungsmessstreifen überwacht werden, oder es kann der Winkel zwischen den Förderschaufeln und der Nabe überwacht werden, wenn die Förderschaufeln im Zuge einer Expansion von der Nabe abgewinkelt werden.

Es kann auch beispielsweise ein elektrischer Widerstand überwacht werden, der einem Strompfad vom Pumpengehäuse über die Förderschaufeln und die Nabe entspricht, wobei im expandierten Zustand die Förderschaufeln bzw. andere Teile des Rotors das Pumpengehäuse nicht berühren sollten und somit der Widerstand hoch ist, während im komprimierten Zustand eine Berührung zwischen dem Pumpengehäuse und anderen Teilen der Pumpe gegeben ist, so dass der elektrische Widerstand in diesem Zustand verringert ist.

Ähnlich kann auch eine elektrische Kapazität zwischen dem Pumpengehäuse und Teilen des Rotors oder dem gesamten Rotor überwacht werden, wobei die Kapazität von dem Abstand zwischen diesen Bauteilen beeinflusst ist.

Es kann auch eine Induktivität des Gehäuses bzw. Rotors überwacht werden, wobei die Induktivität von der Form des Gehäuses bzw. Rotors beeinflusst ist. Wenn das Gehäuse bzw. der Rotor beispielsweise ein Gerüst aus Metall, beispielsweise Nitinol, enthält, welches sich beim Wechsel vom Transportzustand in den Betriebszustand (hyper-)elastisch verformt, so ändert sich abhängig vom Verlauf der elektrisch leitenden Gerüstkomponenten auch die Induktivität des Gehäuses bzw. Rotors. Die Änderung dieser Induktivität kann beispielsweise durch Überwachung des Blindwiderstandes des Gehäuses bzw. Rotors erfasst werden. Auch könnte man beispielweise des Gehäuse mit einem elastisch verformbaren elektrischen Leiter umwickeln, der beim Wechsel vom Transportzustand in den Betriebszustand seinen Widerstand und darüber hinaus, da spulenförmig angeordnet, die Induktivität ändert.

Eine weitere Variante kann vorsehen, dass ein zusätzlicher leitender Pfad, beispielsweise ein Draht oder mehrere einander berührende Drähte, aus elektrisch leitendem Material in die Pumpe eingebracht ist, der durch eine Expansionsbewegung getrennt oder zerrissen, d. h. unterbrochen wird, so dass bei Überwachung des Durchgangswiderstandes des Pfades mit dessen Unterbrechnung eine Änderung eintritt. Ein solcher Draht oder leitender Pfad kann beispielsweise im komprimierten Zustand der Pumpe diese komplett umgeben und um diese herum angeordnet, insbesondere gespannt sein.

Es kann auch vorgesehen sein, dass der überwachte Pfad, der die Pumpe oder Elemente der Pumpe umgibt und im komprimierten Zustand nicht leitend ist, erst durch die Expansion durch Verschiebung eines Leiters leitend wird.

Zur Übertragung des Signals vom Pumpenkopf zum proximalen Katheterabschnitt kann beispielsweise ein elektrischer Leiter in den Katheter integriert werden.

In diesem Sinne wird als eigene Erfindung auch eine Pumpeneinrichtung mit den folgenden Merkmalen vorgeschlagen:
Pumpeneinrichtung mit einem Pumpenkopf und einem Katheter, wobei der Katheter ein außerhalb eines menschlichen oder tierischen Körpers platzierbares proximales Ende und ein zum Pumpenkopf hin befindliches distales Ende aufweist, wobei der Pumpenkopf ein mechanisch bewegliches Pumpglied aufweist und eine von dem proximalen zu dem distalen Ende des Katheters verlaufende Energiezuführungseinrichtung zum Antrieb des Pumpglieds in Form einer flexiblen Welle vorgesehen ist, wobei der Katheter einen zusätzlichen elektrischen Leiter aufweist, der von dem proximalen Ende des Katheters zum distalen Ende des Katheters verläuft.

Hiermit ist es erstmals möglich, dass bei einer mechanisch angetriebenen Pumpe zusätzliche elektrische Signale aus dem Pumpenkopf zum am außerhalb des menschlichen/tierischen Körpers befindlichen proximalen Ende, an dem eine medizinische Bedienperson stehen kann, übertragbar sind. Es wird hierdurch möglich, beispielsweise Kontrollfunktionen auszuüben, um eine Sicherstellung der korrekten Funktion zu gewährleisten bzw. zu beobachten.

Eine weitere Ausgestaltung der Erfindung kann vorsehen, dass der Sensor eine Halteeinrichtung überwacht, die die Pumpe komprimiert und die aufweitbar ist oder aus der die Pumpe entfernbar ist.

Als Halteeinrichtung kann beispielsweise das Ende eines Hohlkatheters vorgesehen sein, in dem die Pumpe während eines Transportvorgangs untergebracht ist, wobei die Pumpe durch den sie umgebenden Hohlkatheter komprimiert wird, bis dass sie nach dem Verbringen zum Einsatzort aus dem Hohlkatheter herausgeschoben wird, beispielsweise unter Zuhilfenahme einer Antriebswelle oder eines weiteren Zuges oder Katheterschlauches als Schubwerkzeug. Nachdem die Pumpe aus dem Hohlkatheter herausgeschoben ist, entfällt die komprimierende Haltekraft des Hohlkatheters, so dass die Pumpenelemente elastisch expandieren können. Dieser Vorgang kann zusätzlich durch weitere Maßnahmen wie beispielsweise langsame Drehung des Pumpenrotors oder Manipulation durch zusätzliche Zug- oder Schubelemente unterstützt werden.

Es kann ein Sensor am Ende des Hohlkatheters vorgesehen sein, der signalisiert, dass die Pumpe aus dem Hohlkatheter vollständig herausgeschoben worden ist. Damit ist mit einer gewissen Sicherheit gegeben, dass die Pumpe sich selbsttätig entfaltet hat.

Es können auch verschiedene der genannten und oben beschriebenen Sensoren miteinander kombiniert und logisch verschaltet werden, um eine höhere Sicherheit darüber zu gewinnen, ob die Pumpe tatsächlich expandiert worden ist, bevor sie in Betrieb genommen wird. Im Folgenden wird die Erfindung anhand eines Ausführungsbeispiels in einer Zeichnung gezeigt und anschließend beschrieben. Dabei zeigt
- Fig. 1: schematisch eine Herzkatheterpumpe beim Durchschieben durch ein Blutgefäß in eine Herzkammer,
- Fig. 2: die Pumpe aus Fig. 1 nach dem Erreichen der Herzkammer und dem Expandieren,
- Fig. 3: eine erste Signalverarbeitung von Detektor-signalen,
- Fig. 4: eine zweite Verarbeitung von Detektor-signalen,
- Fig. 5: eine Verarbeitung von Detektorsignalen mit zusätzlicher Steuerung der Antriebseinrichtung,
- Fig. 6: eine Signalverarbeitung, bei der fallweise die Verbindung zwischen der Antriebseinrichtung und der Pumpe unterbrochen wird,
- Fig. 7: eine Detektionseinrichtung, die an der Antriebseinrichtung angeordnet ist,
- Fig. 8: eine Pumpe in komprimierter Form,
- Fig. 9: die Pumpe aus Fig. 8 in expandierter Form,
- Fig. 10: eine Pumpe, die in komprimierter Form in einem Herzkatheter positioniert ist,
- Fig. 11: eine Detektionseinrichtung einer Pumpe, die einen elektrischen Widerstand misst,
- Fig. 12: eine Detektionseinrichtung, die den Widerstand eines eine Pumpe umspannenden Drahtes misst,
- Fig. 13: eine Detektionseinrichtung ähnlich der aus Fig. 12 mit einem unterbrochenen Leiter.
- Fig. 14: eine Pumpe im komprimierten Zustand, umgeben von einem nicht leitenden Pfad,
- Fig. 15: die Pumpe aus Fig. 14 im wenigstens teilweise expandierten Zustand mit geschlossenem Leiterpfad,
- Fig. 16: eine Pumpe schematisch in einem Längsschnitt, während sie am Ende eines Hohlkatheters in diesem komprimiert ist und
- Fig. 17: die Pumpe aus Fig. 16 außerhalb des Katheters im expandierten Zustand.

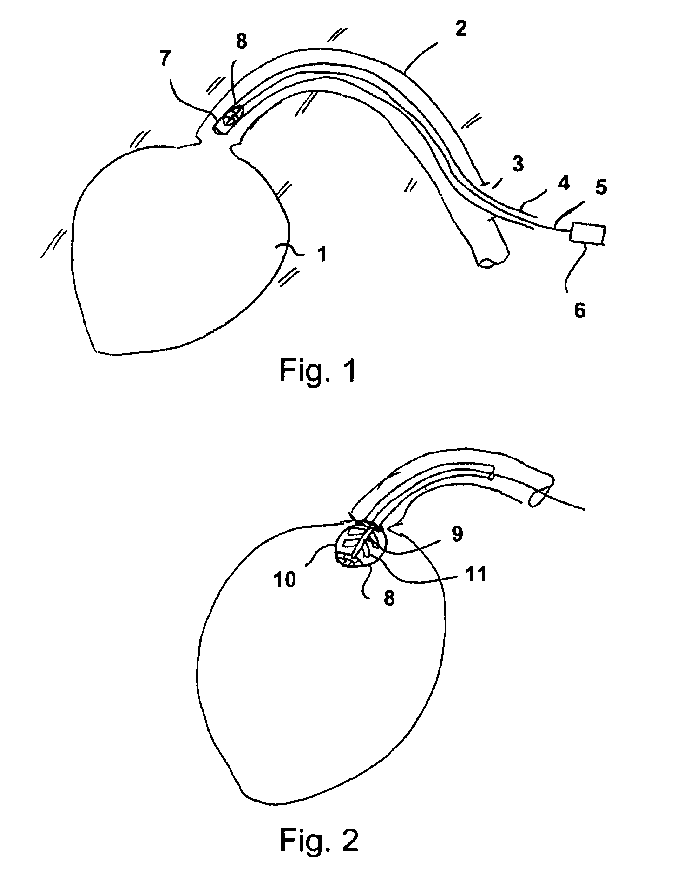

Fig. 1 zeigt schematisch eine Herzkammer 1, in die ein Blutgefäß 2 mündet, durch das mittels einer Schleuse 3 ein Hohlkatheter 4 eingeführt ist. Der Hohlkatheter weist in seinem Hohlraum eine rotierende Welle 5 auf, die mit hohen Umdrehungszahlen, typischerweise mehr als 10.000 Umdrehungen pro Minute, mittels eines Motors 6 antreibbar ist.

Die Welle 5 treibt am distalen Ende 7 des Hohlkatheters 4 eine Pumpe 8 an, die in der Darstellung der Fig. 1 noch in komprimiertem Zustand am Ende des Hohlkatheters durch diesen komprimiert ist.

Die Pumpe kann beispielsweise mittels der Welle 5 oder weiterer nicht dargestellter Elemente aus dem Ende des Hohlkatheters 4 in die Herzkammer 1 eingeschoben werden. Der damit entstehende Zustand ist in der Fig. 2 dargestellt, wo die Pumpe 8 in expandierter Form gezeigt ist. Dabei ist ein Rotor 9 im Inneren eines Pumpengehäuses 10 in schematischer Form gezeigt.

Der Rotor 9 weist radial von einer Nabe abstehende Förderschaufeln 11 auf, die im komprimierten Zustand der Pumpe eingerollt, eingeklappt oder anderweitig komprimiert sind.

In der in der Fig. 2 dargestellten Gestalt ist die Pumpe 8 betriebsfähig, d. h., sie befindet sich im Betriebszustand und kann durch Rotation des Rotors Blut fördern. In der Fig. 1 ist dagegen die Pumpe 8 im Transportzustand gezeigt, d. h., sie ist innerhalb des Hohlkatheters 4 komprimiert.

Der Übergang von der komprimierten Form der Pumpe zur expandierten Form kann beispielsweise bei Wegfall der äußeren Kompressionskräfte durch Eigenelastizität des Pumpengehäuses 10 und des Rotors 9 erfolgen.

Es können jedoch auch zusätzliche Manipulationselemente vorgesehen sein, wie beispielsweise Züge, die entlang des Hohlkatheters 4 an dessen Außenseite oder im Lumen verlaufen und die durch Aufbringen von Zug oder Druck auf die Pumpe deren Aufklappen und Entfalten bewirken können.

Letztlich kann die Pumpe auch beispielsweise dadurch expandiert werden, dass der Rotor langsam gedreht wird, so dass beispielsweise durch den Fluidgegendruck des Blutes, das sich in den Förderschaufeln 11 fängt, diese aufgerichtet werden.

Das Pumpengehäuse 10 kann durch einen leichten Überdruck, der durch den Rotor erzeugt wird, ebenfalls aufgepumpt werden.

Es ist auch denkbar, die Pumpe mit aufpumpbaren Hohlräumen auszustatten, indem beispielsweise das Pumpengehäuse 10 als doppelwandiger Ballon hergestellt wird und der Rotor ebenfalls aufpumpbare Hohlräume sowohl in den Förderschaufeln als auch gegebenenfalls in der Nabe aufweist, wobei die einzelnen Elemente der Pumpe in diesem Fall über hydraulische oder pneumatische Leitungen mit einer steuerbaren Druckquelle verbunden sein müssen. Die genannten Bauteile können auch aus einem Schaumstoff bestehen, der seine expandierte Form nach Wegfall von Kompressionskräften selbsttätig einnimmt.

Maßgebend für eine erhöhte Betriebssicherheit ist dabei, dass der Entfaltungszustand der Pumpe auch geprüft und nachgewiesen wird, so dass beispielsweise ein behandelnder Arzt entscheiden kann, ob die Pumpe in Betrieb genommen werden kann.

Für diesen Zweck können verschiedenartige Sensoren vorgesehen werden, die weiter unten noch näher beschrieben werden.

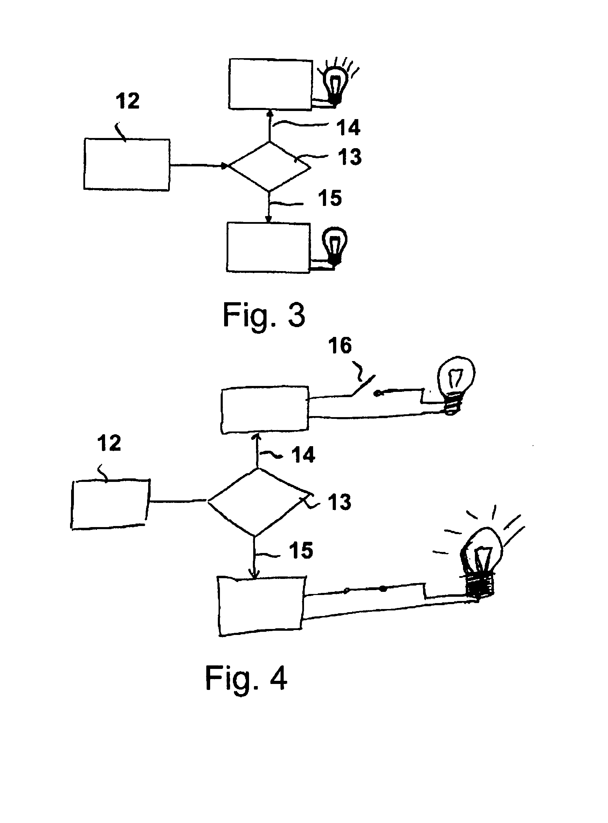

Anhand der Fig. 3 ist dargestellt, wie ein von einem derartigen Sensor kommendes Signal verarbeitet werden kann.

Der Sensor ist in der Fig. 3 mit 12 bezeichnet. Er liefert ein Signal an eine Entscheidungseinrichtung 13, die aufgrund der Sensorsignale entscheidet, ob die Pumpe entfaltet ist oder nicht oder ob ein bestimmtes Element der Pumpe entfaltet ist. Ist der Entfaltungszustand erreicht (ja), so wird der Signalweg 14 gewählt. Ist der Entfaltungszustand nicht erreicht (nein), so wird das Signal 15 abgesetzt. In Abhängigkeit davon, wie der Zustand der Pumpe ist, wird ein Signal, in der Fig. 3 beispielsweise ein Lichtsignal, ausgegeben, wenn der Betriebszustand erreicht ist, oder unterdrückt, solange der Betriebszustand nicht erreicht ist.

Die Lichtsignalzuordnung kann auch genau umgekehrt sein, so dass ein Lichtsignal abgegeben wird, solange der Betriebszustand nicht erreicht ist, und dieses erlischt, sobald der Betriebszustand erreicht ist.

Anstelle von Lichtsignalen können auch andersartige Signale wie beispielsweise akustische Signale ausgesandt werden.

In der Fig. 4 ist dargestellt, dass nach derselben Struktur wie in der Fig. 3 bei Erreichen des Betriebszustandes und entsprechendem Einnehmen des Signalzustandes 14 (ja) ein Schalter 16 zu einem Signalelement unterbrochen wird, während dasselbe oder auch ein anderes Schaltelement beim Einnehmen des Signalzustandes 15 (nein) geschlossen wird.

An die entsprechenden Schalter 16 können anstelle eines Signalelementes auch andere Elemente angeschlossen werden, die bestimmte gewünschte Wirkungen erzielen.

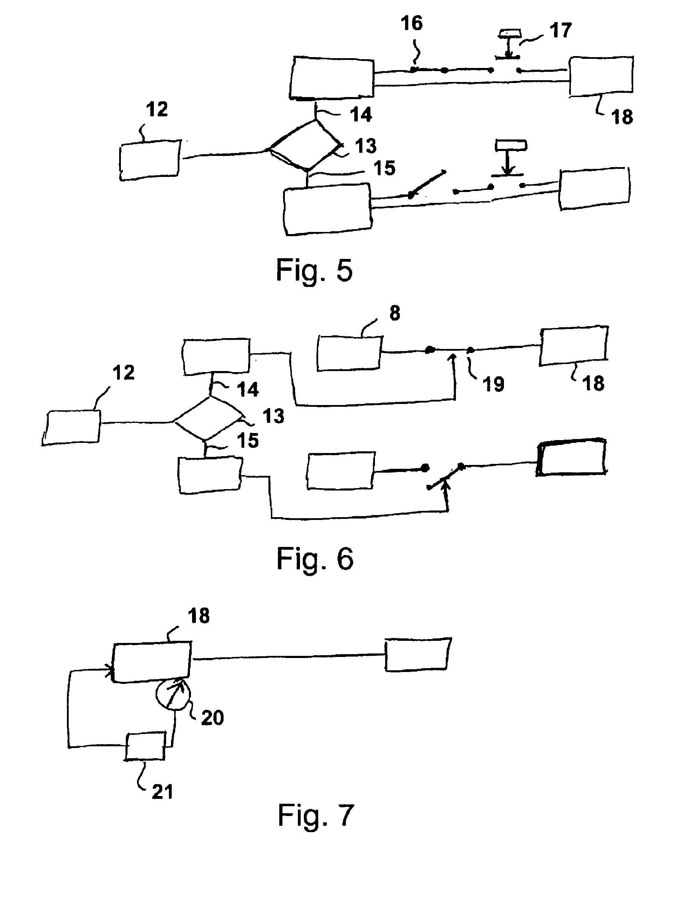

In der Fig. 5 ist eine Konstellation dargestellt, in der in Reihe mit einem Schalter 16, der signalabhängig betätigt wird, ein weiterer von Hand betätigbarer Schalter 17 vorgesehen ist, der zur Betätigung des Antriebs 18 der Pumpe führt. Dies hat zur Folge, dass im Falle, dass der Betriebszustand erreicht ist (ja), das Signal 14 ausgegeben und der Schalter 16 geschlossen wird. Damit kann durch Betätigen des Schalters 17 der Antrieb eingeschaltet werden.

Solange das Signal für das Erreichen des Betriebszustandes nicht gegeben wird (nein), bezeichnet durch das Signal 15, bleibt der Schalter 16, wie in der unteren Hälfte der Fig. 5 gezeigt, geöffnet, so dass der Schalter 17 zwar betätigt werden kann, jedoch dadurch der Antrieb 18 nicht in Gang gesetzt wird. Mittels des Sensors 12 bzw. seiner Signalverarbeitung wird damit der Antrieb 18 blockiert.

In der Fig. 6 ist eine Variante der in Fig. 5 dargestellten Schaltung beschrieben, bei der durch die Signalverarbeitung ein Schalter 19 betätigt wird, der die Übertragung von Energie vom Antrieb 18 zur Pumpe 8 zulässt oder sperrt. Ist der Betriebszustand der Pumpe erreicht, gekennzeichnet durch das Signal 14, so wird der Schalter 19 geschlossen, und die Energie kann vom Antrieb 18 zur Pumpe gelangen. Solange der Betriebszustand nicht erreicht ist, gekennzeichnet durch das Signal 15, bleibt der Schalter 19 geöffnet, und die Wirkung des Antriebs erreicht die Pumpe nicht.

Die Beispiele für eine Antriebssteuerung der Fig. 5 und 6 können auch derart mit einer Signalverarbeitung entsprechend den Beispielen der Fig. 1 bis 4 kombiniert werden dass sowohl ein außerhalb des Körpers wahrnehmbares Signal erscheint bzw. erlischt, als auch die Energiezufuhr der Antriebseinrichtung freigeschaltet bzw. gesperrt wird.

Der Schalter 19 kann beispielsweise als elektrischer Schalter, jedoch auch als pneumatisches oder hydraulisches Ventil ausgebildet sein, das im Antriebsstrang angeordnet ist.

Es kann sich auch um eine Kupplung handeln, die die Übertragung des Drehmomentes über die Welle dadurch blockiert, dass die Kupplung in den Auskupplungszustand wechselt, in dem kein Drehmoment übertragen wird.

Fig. 7 zeigt schematisch die Anordnung eines Sensors 20 direkt am Antrieb 18, wobei der Sensor 20 beispielsweise die den Antrieb durchfließende Stromstärke oder in anderer Weise die Last des Antriebs misst.

Ist die Pumpe noch nicht im Betriebszustand, so wird die Last des Antriebs höher als im Betriebszustand, da die Pumpe sich entweder überhaupt nicht oder nur unter großen Reibungsverlusten bewegen lassen wird.

Die Last wird vom Sensor 20 erfasst und mittels einer Verarbeitungseinrichtung 21 als Teil der Detektionseinrichtung mit Referenzwerten oder Referenzmustern verglichen, wobei die Einrichtung 21, wenn festgestellt wird, dass der Betriebszustand noch nicht erreicht ist, den Antrieb 18 beeinflussen, beispielsweise abschalten kann.

Der Sensor 20 ist in diesem Fall typischerweise von der Pumpe entfernt und eher antriebsnah, d. h. bei dem in den Fign. 1 und 2 gezeigten Beispiel in der Nähe des Elektromotors 6 oder seiner Stromquelle, angeordnet. Dabei kann auch vorgesehen sein, dass der entsprechende Motor, entweder ein elektrischer Motor oder beispielsweise auch eine Mikroturbine, am distalen Ende des Hohlkatheters 4 angeordnet sein kann, so dass in diesem Fall auch der Sensor 20 dort angeordnet ist.

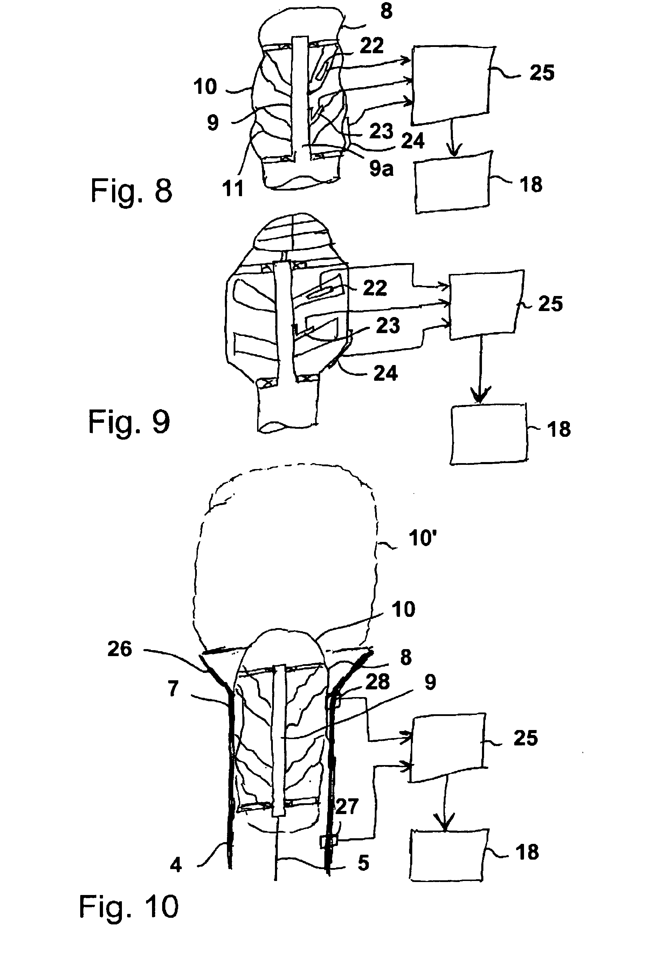

In der Fig. 8 ist schematisch eine Pumpe 8 im komprimierten Zustand mit einem zusammengefallenen Gehäuse 10 und einem Rotor 9 dargestellt, dessen Förderschaufeln 11 ebenfalls komprimiert, beispielsweise an die Nabe 9a angeklappt sind.

Es sind Dehnungsmessstreifen 22, 23, 24 dargestellt, wobei der Dehnungsmessstreifen 22 auf einer Förderschaufel angeordnet ist, der Dehnungsmessstreifen 23 im Ansatzbereich einer Förderschaufel an die Nabe 9a und der Dehnungsmessstreifen 24 außen am Gehäuse 10.

Die Dehnungsmessstreifen sind jeweils aufgeklebt und registrieren Form- und Größenänderungen des Trägermaterials. Sie sind jeweils über Leitungen mit einer Auswerteeinrichtung 25 verbunden, die das Ausmaß von Formänderungen während des Expansionsprozesses der Pumpe registriert. Die Signale der einzelnen Dehnungsmessstreifen können kombiniert werden, wobei die Logik dieser Kombination verschieden ausgestaltet sein kann. Beispielsweise kann das Erreichen des Betriebszustandes erst dann signalisiert werden, wenn alle Messstreifen eine Expansion des jeweils von ihnen überwachten Elementes melden oder wenn wenigstens zwei dieser Elemente oder auch schon ein einziges Element das Erreichen des Expansionszustandes meldet. Entsprechend wird ein Signal an den Antrieb 18 abgegeben.

In der Fig. 9 ist die Pumpe aus der Fig. 8 in expandiertem Zustand dargestellt.

Die Fig. 10 zeigt das Ende 7 eines Hohlkatheters 4, in dem eine Pumpe 8 komprimiert ist. Die Pumpe 8 ist mittels der Welle 5 durch den Hohlkatheter 4 mit dem Motor 6 verbunden. Sowohl das Gehäuse 10 als auch der Rotor 9 mit den Förderschaufeln sind komprimiert.

Gestrichelt ist das Gehäuse 10' in einem Zustand dargestellt, der nach der Expansion der Pumpe erreicht wird. Es ist zudem ein Einführkonus 26 dargestellt, über den die Pumpe 10 beim Einziehen in den Hohlkatheter 4 vor dem Entfernen aus dem Patientenkörper eingefahren und dort radial komprimiert wird.

Es sind innerhalb des Hohlkatheters 4 zwei Sensoren 27, 28 vorgesehen, die signalisieren, ob die Pumpe in dem von ihnen repräsentierten und überwachten Bereich des Hohlkatheters steckt oder aus diesem schon ausgefahren ist. In der Darstellung der Fig. 10 ist der Bereich des Sensors 27 durch die Pumpe bereits passiert worden, jedoch signalisiert der Sensor 28 noch, dass die Pumpe in diesem Bereich komprimiert ist. Nach dem Ausfahren aus dem Hohlkatheter wird der Sensor 28 melden, dass er freiliegt und dass die Pumpe aus dem Hohlkatheter ausgefahren und damit expandiert ist. Die entsprechenden Signale werden in einer Signalverarbeitungseinrichtung 25 bearbeitet und an den Antrieb 18 gegebenenfalls weitergegeben.

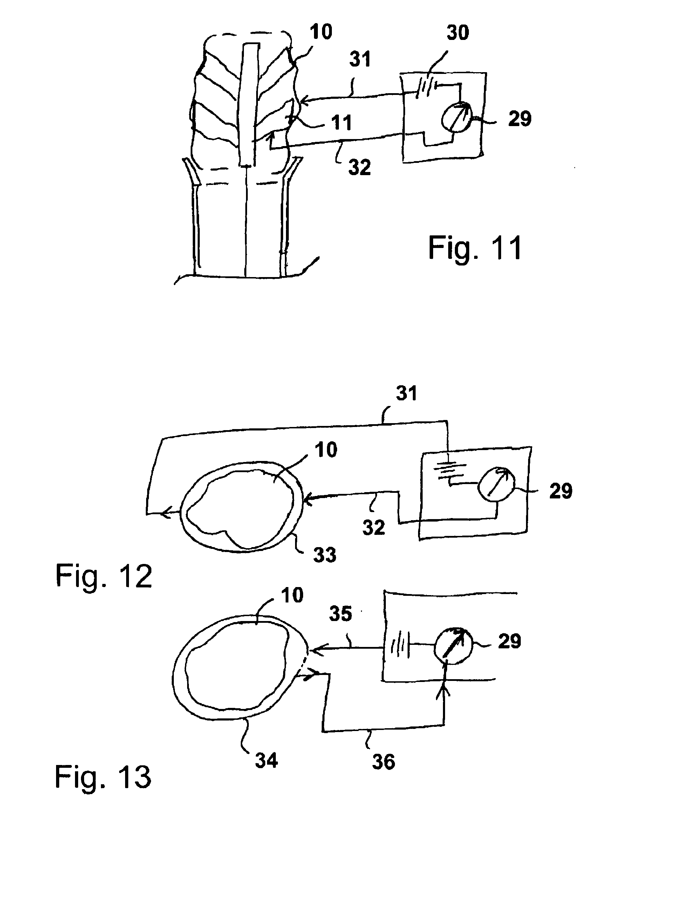

Fig. 11 zeigt einen Sensor 29, der als Widerstandsmesssensor ausgeführt ist und der mit einer Stromquelle 30 in Reihe geschaltet und über zwei Leitungen 31, 32 mit dem Pumpengehäuse 10 und mit einer Förderschaufel 11 verbunden ist. Sowohl das Gehäuse 10 als auch die Förderschaufel können beispielsweise aus einem wenigstens teilweise leitenden Material bestehen oder mit einem solchen Material beschichtet sein, so dass der Stromkreis über die Leitungen 31, 32 über den Sensor 29 und zwischen der Förderschaufel 11 und dem Pumpengehäuse 10 geschlossen ist, sobald die Förderschaufel 11 das Gehäuse 10 berührt.

In diesem Fall würde signalisiert, dass die Pumpe sich nicht im Betriebszustand befindet.

Sobald der Kontakt zwischen der Förderschaufel 11 und dem Gehäuse 10 aufgehoben wird, signalisiert die Detektionseinrichtung das Erreichen des Betriebszustands.

Fig. 12 stellt eine Widerstandsüberwachung eines geschlossenen Drahtrings 33 dar, der eine komprimierte Pumpe 10 umspannt. Reißt der Draht bei Expansion der Pumpe, so verdoppelt sich der Widerstand im Ring, da einer der Strompfade unterbrochen ist. Dies wird durch den Sensor 29 registriert und entsprechend der Betriebszustand signalisiert.

Fig. 13 zeigt eine ähnliche Einrichtung wie Fig. 12 mit einem zwischen den Zuleitungen 35, 36 unterbrochenen Ringleiter 34, der beispielsweise in einen geschlossenen Isolierring integriert ist. Bricht der Isolierring an einer Stelle, an der Ringleiter 34 verläuft, so wird der Strompfad durch den Ringleiter unterbrochen, was durch den Sensor 29 registriert wird.

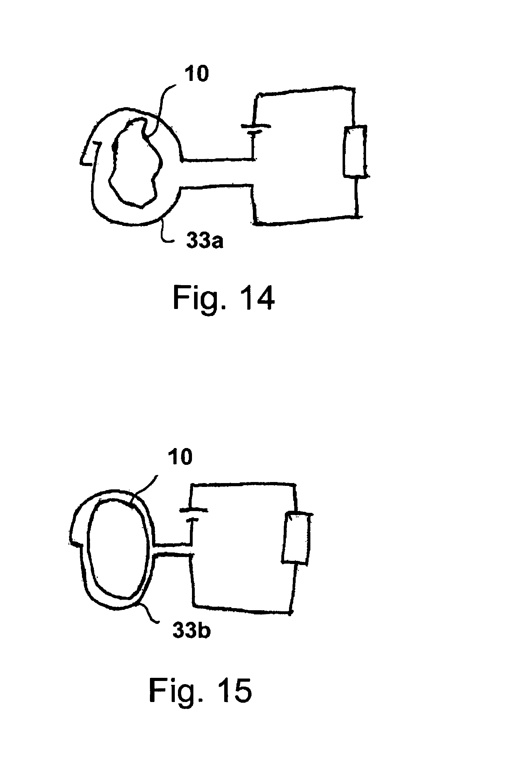

Fig. 14 zeigt eine ähnliche Vorrichtung wie Fig. 13 mit einer komprimierten Pumpe 10, wobei jedoch im Unterschied zu Fig. 13 der Stromkreis des Ringleiters 33a zunächst im Transportzustand offen ist und im entfalteten Zustand der Pumpe, also dem Betriebszustand, geschlossen wird. Der Vorteil dieser Variante liegt darin begründet, dass der Betriebszustand nur dann angezeigt wird, wenn der Ringleiter selbst keinen Defekt aufweist. In der Ausführung nach Fig. 13 würde ein defekter, unbeabsichtigt unterbrochener Ringleiter eventuell auch dann das Erreichen des Betriebszustandes anzeigen, wenn dieser (noch) nicht erreicht wäre. In einer Ausführung nach Fig. 14 wäre dies nicht möglich, was eine zusätzliche Sicherheit des Systems gegen Fehlfunktion bietet.

Fig. 15 zeigt die Pumpe 10 mit der Vorrichtung aus Fig. 14 im entfalteten Zustand (Betriebszustand) mit geschlossenem Ringleiter 33b.

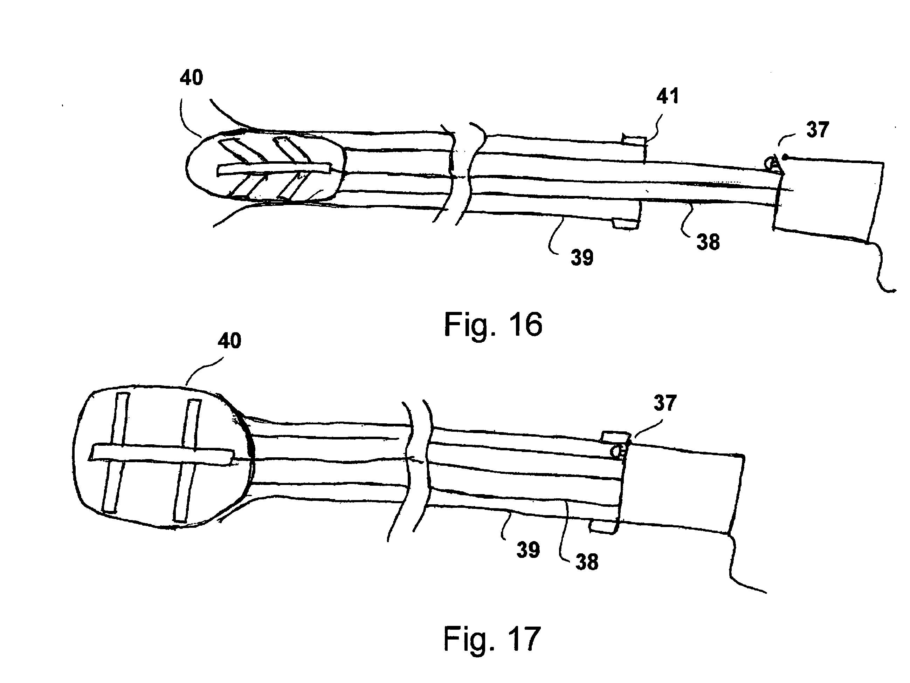

Fig. 16 zeigt eine Vorrichtung ähnlich der in Fig. 10 im Transportzustand,aber so ausgebildet, dass am proximalen Ende des Katheters 38 ein Schalter 37 angeordnet ist. Dabei ist die Länge des Katheters 38 im Verhältnis zur Länge des Hohlkatheters 39 so bemessen, dass der Pumpenkopf 40 bereits vollständig den Betriebszustand eingenommen hat (also den Hohlkatheter 39 verlassen hat), wenn das proximale Ende 41 des Hohlkatheters 39 den Schalter 37 betätigt (vgl. Fig. 17). Zur Vermeidung ungewollten Auslösens des Schalters 37 ist dieser vorteilhaft so ausgebildet bzw. angeordnet, dass ein Auslösen des Schalters mit den Händen während der Manipulation nicht möglich ist. Der Schalter 37 kann bei ausreichender Länge der Katheter 38,39 außerhalb eines Patientenkörpers angeordnet sein.

Fig. 17 zeigt die Vorrichtung aus Fig. 16 im Betriebszustand.

Die beschriebene Erfindung dient dazu, die Betriebssicherheit von komprimierbaren Pumpen zu erhöhen und besonders im medizinischen Bereich Gesundheitsrisiken zu senken.

## Patentansprüche

1. Pumpeneinrichtung mit einer Pumpe (8) und einer Energiezuführungseinrichtung (5, 18), wobei die Pumpe ein Förderelement (9, 11) aufweist, das mittels zugeführter Energie ein Fluid fördert, wobei die Pumpe einen Transportzustand und einen Betriebszustand aufweist und wobei wenigstens ein erstes Element (9, 9a, 10, 10', 11) der Pumpe im Transportzustand eine andere Form und/oder Größe aufweist als im Betriebszustand, **gekennzeichnet durch** eine Detektionseinrichtung (12, 20, 21, 22, 23, 24, 25, 27, 28, 29), die erfasst, ob wenigstens das erste Element sich bezüglich Form und/oder Größe im Betriebszustand befindet.

2. Pumpeneinrichtung nach Anspruch 1, **dadurch gekennzeichnet, dass** die Detektionseinrichtung(12, 20, 21, 22, 23, 24, 25, 27, 28, 29) einen Sensor aufweist.

3. Pumpeneinrichtung nach Anspruch 1 oder 2, **dadurch gekennzeichnet, dass** die Energiezuführungseinrichtung (5, 18) ein mechanischer Antrieb ist und dass das Förderelement (9, 11) beweglich und antreibbar ist.

4. Pumpeneinrichtung nach Anspruch 1,2 oder 3, **dadurch gekennzeichnet, dass** das Förderelement einen Rotor (9) mit wenigstens einer Förderschaufel (11) aufweist.

5. Pumpeneinrichtung nach Anspruch 1,2 oder 3, **dadurch gekennzeichnet, dass** das Förderelement (9, 11) ein aufpumpbares Ballonelement aufweist.

6. Pumpeneinrichtung nach Anspruch 1 oder einem der folgenden, **dadurch gekennzeichnet, dass** das Förderelement (9, 11) aufpumpbar, biegbar, schwenkbar oder elastisch komprimierbar und expandierbar ist.

7. Pumpeneinrichtung nach Anspruch 1 oder einem der folgenden, **dadurch gekennzeichnet, dass** die Pumpe ein komprimierbares und expandierbares Gehäuse (10, 10') aufweist.

8. Pumpeneinrichtung nach Anspruch 1 oder einem der folgenden, **dadurch gekennzeichnet, dass** die Detektionseinrichtung (12, 20, 21, 22, 23, 24, 25, 27, 28, 29) einen Signalzustand ändert, wenn das erste Element den Betriebszustand erreicht.

9. Pumpeneinrichtung nach Anspruch 7, **dadurch gekennzeichnet, dass** die Detektionseinrichtung (12, 20, 21, 22, 23, 24, 25, 27, 28, 29) ein Signal abgibt, solange der Betriebszustand nicht erreicht ist.

10. Pumpeneinrichtung nach Anspruch 7, **dadurch gekennzeichnet, dass** die Detektionseinrichtung (12, 20, 21, 22, 23, 24, 25, 27, 28, 29) ein Signal abgibt, solange der Betriebszustand erreicht ist.

11. Pumpeneinrichtung nach Anspruch 1 oder einem der folgenden, **dadurch gekennzeichnet, dass** die Detektionseinrichtung (12, 20, 21, 22, 23, 24, 25, 27, 28, 29) die Energiezuführungseinrichtung (18) blockiert, solange der Betriebszustand nicht erreicht ist.

12. Pumpeneinrichtung nach Anspruch 1 oder einem der folgenden, **dadurch gekennzeichnet, dass** die Detektionseinrichtung (12, 20, 21, 22, 23, 24, 25, 27, 28, 29) im Betrieb die Last der Energiezuführungseinrichtung (18) überwacht und durch Vergleich mit einem Referenzwert oder einem Referenzmuster ermittelt, ob der Betriebszustand erreicht ist.

13. Pumpeneinrichtung nach Anspruch 1 oder einem der folgenden, **dadurch gekennzeichnet, dass** die Detektionseinrichtung (12, 20, 21, 22, 23, 24, 25, 27, 28, 29) einen Sensor (20) aufweist, der unmittelbar an der Pumpe (8) angeordnet ist.

14. Pumpeneinrichtung nach Anspruch 13, **dadurch gekennzeichnet, dass** der Sensor (27, 28, 29) den Betriebszustand unabhängig vom Betrieb der Pumpe (8) erfasst.

15. Pumpeneinrichtung nach einem der Ansprüche 1 bis 14, **dadurch gekennzeichnet, dass** der Sensor (22, 23, 24, 27, 28) ein Positionssensor ist.

16. Pumpeneinrichtung nach einem der Ansprüche 1 bis 15, **dadurch gekennzeichnet, dass** der Sensor (27, 28, 29) eine Halteeinrichtung (4, 33, 34) überwacht, die die Pumpe (8) komprimiert und die aufweitbar ist oder aus der die Pumpe entfernbar ist.

17. Pumpe nach einem der Ansprüche 1 bis 16 **dadurch gekennzeichnet, dass** wenigstens ein erstes Element (9, 10, 10', 11)wenigstens teilweise aus einem elastisch verformbaren, insbesondere komprimierbaren und expandierbaren Material besteht.

18. Pumpe nach einem der Ansprüche 1 bis 17, **dadurch gekennzeichnet, dass** wenigstens ein erstes Element (9, 10, 10', 11)den Wechsel vom Transportzustand in den Betriebszustand durch elastische Verformung vollzieht.

19. Pumpeneinrichtung mit einem Pumpenkopf und einem Katheter, wobei der Katheter ein außerhalb eines menschlichen oder tierischen Körpers platzierbares proximales Ende und ein zum Pumpenkopf hin befindliches distales Ende aufweist, wobei der Pumpenkopf ein mechanisch bewegliches Pumpglied aufweist und eine von dem proximalen zu dem distalen Ende des Katheters verlaufende Energiezuführungseinrichtung zum Antrieb des Pumpglieds in Form einer flexiblen Welle vorgesehen ist, **dadurch gekennzeichnet, dass** der Katheter einen zusätzlichen elektrischen Leiter aufweist, der von dem proximalen Ende des Katheters zum distalen Ende des Katheters verläuft.
